# EUROPEAN PATENT SPECIFICATION

(11) **EP 1 009 400 B1**
(45) Date of publication and mention of the grant of the patent: **29.12.2004**
(21) Application number: 98935250.5
(22) Date of filing: 11.08.1998
(51) Int. Cl.: A61K 31/40, A61K 31/00

(54) **COMBINATION THERAPY COMPRISING ATORVASTATIN AND AN ANTIHYPERTENSIVE AGENT**
KOMBINATIONSTHERAPIE, ENTHALTEND ATORVASTATIN UND BLUDDRUCKSENKENDES MITTEL
THERAPIE COMBINEE UTILISANT DE L'ATORVASTATINE ET UN ANTIHYPERTENSEUR

(30) Priority: 29.08.1997 US 57276 P
(43) Date of publication of application: 21.06.2000
(60) Divisional application: 04023093.0
(73) Proprietor: PFIZER INC., New York, N.Y. 10017 (US)
(72) Inventor: SCOTT, Robert, Andrew, Donald, Riverside, CT 06878 (US)
(74) Representative: McMunn, Watson Palmer
(86) International application number: PCT/IB1998/001230
(87) International publication number: WO 1999/011260

(56) References cited:
- EP-A- 0 753 298
- WO-A-97/16184
- US-A- 5 543 542
- GANOTAKIS E.S., ET AL.: "Cardiovascular risk factor management-new drugs will provide a bright future." JOURNAL OF DRUG DEVELOPMENT AND CLINICAL PRACTICE, vol. 8, no. 2, September 1996, pages 57-60, XP002084032

## Description

This invention relates to pharmaceutical combinations of atorvastatin or a pharmaceutically acceptable salt thereof and alpha-adrenergic receptor blocker or diuretic agents, kits containing such combinations and methods of using such combinations to treat subjects suffering from angina pectoris, atherosclerosis, combined hypertension and hyperlipidemia and to treat subjects presenting with symptoms of cardiac risk, including humans. This invention also relates to additive and synergistic combinations of atorvastatin or a pharmaceutically acceptable salt thereof and alpha-adrenergic receptor blocker or diuretic agents whereby those additive and synergistic combinations are useful in treating subjects suffering from angina pectoris, atherosclerosis, combined hypertension and hyperlipidemia and those subjects presenting with symptoms or signs of cardiac risk, including humans.

### BACKGROUND OF THE INVENTION

The conversion of 3-hydroxy-3-methylglutaryl-coenzyme A (HMG-CoA) to mevalonate is an early and rate-limiting step in the cholesterol biosynthetic pathway. This step is catalyzed by the enzyme HMG-CoA reductase. Statins inhibit HMG-CoA reductase from catalyzing this conversion. As such, statins are collectively potent lipid lowering agents.

Atorvastatin calcium, disclosed in U.S. Patent No. 5,273,995 is currently sold as Lipitor® and has the formula

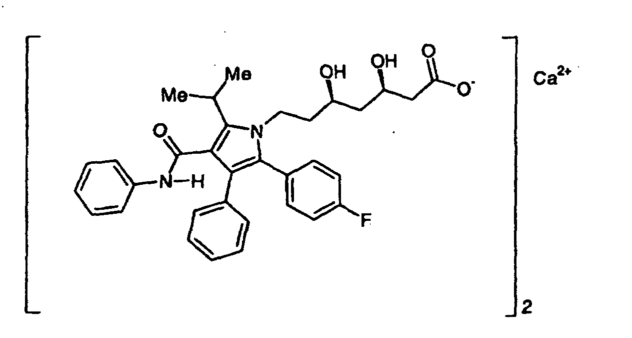

Atorvastatin calcium is a selective, competive inhibitor of MG-CoA. As such, atorvastatin calcium is a potent lipid lowering compound. The free carboxylic acid form of atorvastatin exists predominantly as the lactone of the formula: 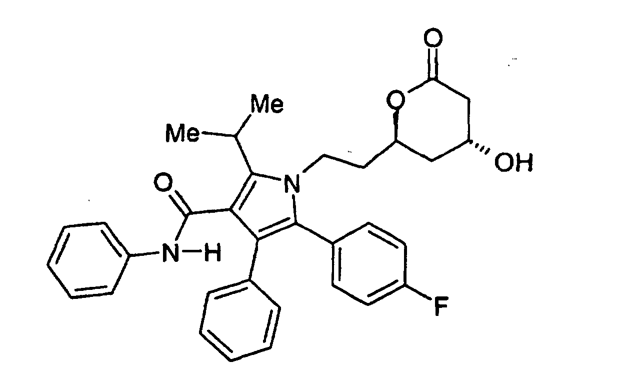 and is disclosed in U.S. Patent No. 4,681,893.

Atherosclerosis is a condition characterized by irregularly distributed lipid deposits in the intima of arteries, including coronary, carotid and peripheral arteries. Atherosclerotic coronary heart disease (hereinafter termed "CHD") accounts for 53% of all deaths attributable to a cardiovascular event. CHD accounts for nearly one-half (about $50-60 billion) of the total U.S. cardiovascular healthcare expenditures and about 6% of the overall national medical bill each year. Despite attempts to modify secondary risk factors such as, *inter alia*, smoking, obesity and lack of exercise, and treatment of dyslipidemia with dietary modification and drug therapy, CHD remains the most common cause of death in the United States.

High levels of blood cholesterol and blood lipids are conditions involved in the onset of atherosclerosis. It is well known that inhibitors of 3-hydroxy-3-methylglutaryl-coenzyme A reductase (HMG-CoA reductase) are effective in lowering the level of blood plasma cholesterol, especially low density lipoprotein cholesterol (LDL-C), in man (Brown and Goldstein, New England Journal of Medicine, 1981, 305, No. 9, 515-517). It has now been established that lowering LDL-C levels affords protection from coronary heart disease (see, e.g., The Scandinavian Simvastatin Survival Study Group: Randomised trial of cholesterol lowering in 4444 patients with coronary heart disease: the Scandinavian Simvastatin Survival Study (4S), Lancet, 1994, 344, 1383-89; and Shepherd, J. et al., Prevention of coronary heart disease with pravastatin in men with hypercholesterolemia, New England Journal of Medicine, 1995, 333, 1301-07).

Angina pectoris is a severe constricting pain in the chest, often radiating from the precordium to the left shoulder and down the left arm. Often angina pectoris is due to ischemia of the heart and is usually caused by coronary disease.

Currently the treatment of symptomatic angina pectoris varies significantly from country to country. In the U.S., patients who present with symptomatic, stable angina pectoris are frequently treated with surgical procedures or PTCA. Patients who undergo PTCA or other surgical procedures designed to treat angina pectoris frequently experience complications such as restenosis. This restenosis may be manifested either as a short term proliferative response to angioplasty-induced trauma or as long term progression of the atherosclerotic process in both graft vessels and angioplastied segments.

The symptomatic management of angina pectoris involves the use of a number of drugs, frequently as a combination of two or more of the following classes: beta blockers, nitrates and calcium channel blockers. Most, if not all, of these patients require therapy with a lipid lowering agent as well. The National Cholesterol Education Program (NCEP) recognizes patients with existing coronary artery disease as a special class requiring aggressive management of raised LDL-C.

Hypertension frequently coexists with hyperlipidemia and both are considered to be major risk factors for developing cardiac disease ultimately resulting in adverse cardiac events. This clustering of risk factors is potentially due to a common mechanism. Further, patient compliance with the management of hypertension is generally better than patient compliance with hyperlipidemia. It would therefore be advantageous for patients to have a single therapy which treats both of these conditions.

Coronary heart disease is a multifactorial disease in which the incidence and severity are affected by the lipid profile, the presence of diabetes and the sex of the subject. Incidence is also affected by smoking and left ventricular hypertrophy which is secondary to hypertension. To meaningfully reduce the risk of coronary heart disease, it is important to manage the entire risk spectrum. For example, hypertension intervention trials have failed to demonstrate full normalization in cardiovascular mortality due to coronary heart disease. Treatment with cholesterol synthesis inhibitors in patients with and without coronary artery disease reduces the risk of cardiovascular morbidity and mortality.

The Framingham Heart Study, an ongoing prospective study of adult men and women, has shown that certain risk factors can be used to predict the development of coronary heart disease. (see Wilson et al., Am. J. Cardiol. **1987, 59(14)**:91G-94G). These factors include age, gender, total cholesterol level, high density lipoprotein (HDL) level, systolic blood pressure, cigarette smoking, glucose intolerance and cardiac enlargement (left ventricular hypertrophy on electrocardiogram, echocardiogram or enlarged heart on chest X-ray). Calculators and computers can easily be programmed using a multivariate logistic function that allows calculation of the conditional probability of cardiovascular events. These determinations, based on experience with 5,209 men and women participating in the Framingham study, estimate coronary artery disease risk over variable periods of follow-up. Modeled incidence rates range from less than 1% to greater than 80% over an arbitrarily selected six year interval. However, these rates are typically less than 10% and rarely exceed 45% in men and 25% in women.

U.S. 4,681,893 discloses that certain statins, including atorvastatin, are hypolipidemic agents and as such are useful in treating atherosclerosis.

### SUMMARY OF THE INVENTION

This invention is directed to a pharmaceutical composition, comprising:
a. an amount of atorvastatin or a pharmaceutically acceptable salt thereof;
b. an amount of an alpha-adrenergic receptor blocker or a pharmaceutically acceptable salt thereof; and
c. a pharmaceutically acceptable carrier or diluent;

This invention is also more particularly directed to a pharmaceutical composition wherein said alpha-adrenergic receptor blocker is doxazosin, prazosin or trimazosin.

This invention is more particularly directed to a pharmaceutical composition comprising the hemicalcium salt of atorvastatin.

This invention is further directed to a pharmaceutical composition, comprising:
a. an amount of atorvastatin or a pharmaceutically acceptable salt thereof;
b. an amount of a diuretic or a pharmaceutically acceptable salt thereof; and
c. a pharmaceutically acceptable carrier or diluent;
This invention is also more particularly directed to a pharmaceutical composition wherein said diuretic is amiloride or bendroflumethiazide.

This invention is more particularly directed to a pharmaceutical composition comprising the hemicalcium salt of atorvastatin.

This invention is also directed to a pharmaceutical product comprising:
a. an amount of an alpha-adrenergic receptor blocker or a pharmaceutically acceptable salt thereof and a pharmaceutically acceptable carrier or diluent;
b. an amount of atorvastatin or a pharmaceutically acceptable salt thereof, and a pharmaceutically acceptable carrier or diluent;
as a combined preparation for simultaneous or sequential use in treating angina pectoris, atherosclerosis, combined hypertension and hypertipidemia and use in cardiac risk management.
This invention is also directed to a pharmaceutical product comprising:
a. an amount of a diuretic or a pharmaceutical acceptable salt thereof and a pharmaceutically acceptable carrier or diluent;
b. an amount of atorvastatin or a pharmaceutically acceptable salt thereof, and a pharmaceutically acceptable carrier or diluent;
as a combined preparation for simultaneous or sequential use in treating angina pectoris, atherosclerosis, combined hypertension and hyperlipidemia and use in cardiac risk management.
This invention is also more particularly directed to a pharmaceutical product wherein said alpha-adrenergic receptor blocker is doxazosin, prazosin or trimazosin.

This invention is also more particularly directed to a pharmaceutical product wherein said diuretic is amiloride or bendroflumethiazide.

This invention is also directed to a kit for achieving a therapeutic effect in a mammal comprising:
a. an amount of atorvastatin or a pharmaceutically acceptable salt thereof and a pharmaceutically acceptable carrier or diluent in a first unit dosage form;
b. an amount of an alpha-adrenergic receptor blocker or a pharmaceutically acceptable salt thereof and a pharmaceutically acceptable carrier or diluent in a second unit dosage form; and
c. container means for containing said first and second dosage forms.
This invention is also directed to a kit for achieving a therapeutic effect in a mammal comprising:
a. an amount of atorvastatin or a pharmaceutically acceptable salt thereof and a pharmaceutically acceptable carrier or diluent in a first unit dosage form;
b. an amount of a diuretic or a pharmaceutically acceptable salt thereof and a pharmaceutically acceptable carrier or diluent in a second unit dosage form; and
c. container means for containing said first and second dosage forms.

This invention is particularly directed to a kit comprising the hemicalcium salt of atorvastatin.

This invention is still more particularly directed to a kit wherein said therapeutic effect is antianginal; antiatheroscterotic; antihypertensive and hypolipidemic; or is effective for cardiac risk management.

This invention is also directed to a use of:
(a) an amount of a first compound, said first compound being atorvastatin or a pharmaceutically acceptable salt thereof; and
(b) an amount of a second compound, said second compound being an alpha adrenergic receptor blocker or a pharmaceutically acceptable salt thereof;
in the preparation of a medicament for the treatment of angina pectoris, atherosclerosis, combined hypertension and hyperlipidemia and in cardiac risk management.
This invention is also directed to a use of:
(a) an amount of a first compound, said first compound being atorvastatin or a pharmaceutically acceptable salt thereof; and
(b) an amount of a second compound, said second compound being a diuretic or a pharmaceutically acceptable salt thereof;
in the preparation of a medicament for the treatment of angina pectoris, atherosclerosis, combined hypertension and hypertipidemia and in cardiac risk management.

This invention is particularly directed to a use comprising the hemicalcium salt of atorvastatin.

Where used herein, the term "cardiac risk" means the likelihood that a subject will suffer a future adverse cardiac event such as, e.g., myocardial infarction, cardiac arrest, cardiac failure, cardiac ischaemia. Cardiac risk is calculated using the Framingham Risk Equation as set forth above. The term "cardiac risk management" means that the risk of future adverse cardiac events is substantially reduced.

It will be recognized by those skilled in the art that certain of the alpha-adrenergic receptor blocker or diuretic agents which are used in combination with atorvastatin or a pharmaceutically acceptable salt of atorvastatin contain either an acidic moiety or a basic moiety which may be reacted with either a base to form a cationic salt or an acid to form an acid addition salt, respectively. All of the pharmaceutically acceptable salts of the alpha-adrenergic receptor blocker or diuretic agents disclosed herein are within the scope of the combination of this invention.

### DETAILED DESCRIPTION OF THE INVENTION

The pharmaceutical compositions of this invention comprise atorvastatin or a pharmaceutically acceptable salt thereof and an antihypertensive agent, comprising an alpha adrenergic blocker or a diuretic agent, or a pharmaceutically acceptable salt thereof.

Atorvastatin may readily be prepared as described in U.S. Patent No. 4,681,893. The hemicalcium salt of atorvastatin, which is currently sold as Lipitor®, may readily be prepared as described in U.S. Patent No. 5,273,995.

The expression "pharmaceutically acceptable salts" includes both pharmaceutically acceptable acid addition salts and pharmaceutically acceptable cationic salts. The expression "pharmaceutically-acceptable cationic salts" is intended to define but is not limited to such salts as the alkali metal salts, (e.g., sodium and potassium), alkaline earth metal salts (e.g., calcium and magnesium), aluminum salts, ammonium salts, and salts with organic amines such as benzathine (N,N'-dibenzylethytenediamine), choline, diethanolamine, ethylenediamine, meglumine (N-methylglucamine), benethamine (N-benzytphenethylamine), diethylamine, piperazine, tromethamine (2-amino-2-hydroxymethyl-1,3-propanediol) and procaine. The expression "pharmaceutically-acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts.

Besides the hemicalcium salt, other pharmaceutically-acceptable cationic salts of atorvastatin may be readily prepared by reacting the free acid form of atorvastatin with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine and tromethamine. The salt is isolated by concentration to dryness or by addition of a non-solvent. In many cases, salts are preferably prepared by mixing a solution of the acid with a solution of a different salt of the cation (sodium or potassium ethylhexanoate, magnesium oleate), and employing a solvent (e.g., ethyl acetate) from which the desired cationic salt precipitates, or can be otherwise isolated by concentration and/or addition of a non-solvent. The acid addition salts of atorvastatin may be readily prepared by reacting the free base form of atorvastatin with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

The pharmaceutically acceptable acid addition and cationic salts of the alpha-adrenergic receptor blocker and diuretic agents used in the combination of this invention may be prepared in a manner analogous to that described for the preparation of the pharmaceutically acceptable acid addition and cationic salts of atorvastatin, but substituting the desired antihypertensive compound for atorvastatin.

Alpha-adrenergic receptor blockers (alpha- or α-blockers) which are within the scope of this invention include, but are not limited to: amosulalol, which may be prepared as disclosed in U.S. Patent No. 4,217,307; arotinolol, which may be prepared as disposed in U.S. Patent No. 3,932,400; dapiprazole, which may be prepared as disclosed in U.S. Patent No. 4,252,721; doxazosin, which may be prepared as disclosed in U.S. Patent No. 4,188,390; fenspiride, which may be prepared as disclosed in U.S. Patent No. 3,399,192; indoramin, which may be prepared as disclosed in U.S. Patent No. 3,527,761; labetolol, which may be prepared as disclosed above; naftopidil, which may be prepared as disclosed in U.S. Patent No. 3,997,666; nicergoline, which may be prepared as disclosed in U.S. Patent No. 3,228,943; prazosin, which may be prepared as disclosed in U.S. Patent No. 3,511,836; tamsulosin, which may be prepared as disclosed in U.S. Patent No. 4,703,063; tolazoline, which may be prepared as disclosed in U.S. Patent No. 2,161,938; trimazosin, which may be prepared as disclosed in U.S. Patent No. 3,669,968; and yohimbine, which may be isolated from natural sources according to methods well known to those skilled in the art.

The term "diuretic," within the scope of this invention, is meant to include diuretic benzothiadiazine derivatives, diuretic organomercurials, diuretic purines, diuretic steroids, diuretic sulfonamide derivatives, diuretic uracils and other diuretics such as amanozine, which may be prepared as disclosed in Austrian Patent No. 168,063; amiloride, which may be prepared as disclosed in Belgian Patent No. 639,386; arbutin, which may be prepared as disclosed in Tschitschibabin, Annalen, 1930, 479, 303; chlorazanil, which may be prepared as disclosed in Austrian Patent No. 168,063; ethacrynic acid, which may be prepared as disclosed in U.S. Patent No. 3,255,241; etozolin, which may be prepared as disclosed in U.S. Patent No. 3,072,653; hydracarbazine, which may be prepared as disclosed in British Patent No. 856,409; isosorbide, which may be prepared as disclosed in U.S. Patent No. 3,160,641; mannitol; metochalcone, which may be prepared as disclosed in Freudenberg et al., Ber., 1957, 90, 957; muzolimine, which may be prepared as disclosed in U.S. Patent No. 4,018,890; perhexiline, which may be prepared as disclosed above; ticrynafen, which may be prepared as disclosed in U.S. Patent No. 3,758,506; triamterene which may be prepared as disclosed in U.S. Patent No. 3,081,230; and urea.

Diuretic benzothiadiazine derivatives within the scope of this invention include: althiazide, which may be prepared as disclosed in British Patent No. 902,658; bendroflumethiazide, which may be prepared as disclosed in U.S. Patent No. 3,265,573; benzthiazide, McManus et al., 136th Am. Soc. Meeting (Atlantic City, September 1959), Abstract of papers, pp 13-O; benzylhydrochlorothiazide, which may be prepared as disclosed in U.S. Patent No. 3,108,097; buthiazide, which may be prepared as disclosed in British Patent Nos. 861,367 and 885,078; chlorothiazide, which may be prepared as disclosed in U.S. Patent Nos. 2,809,194 and 2,937,169; chlorthalidone, which may be prepared as disclosed in U.S. Patent No. 3,055,904; cyclopenthiazide, which may be prepared as disclosed in Belgian Patent No. 587,225; cyclothiazide, which may be prepared as disclosed in Whitehead et al., Journal of Organic Chemistry, 1961, 26, 2814; epithiazide, which may be prepared as disclosed in U.S. Patent No. 3,009,911; ethiazide, which may be prepared as disclosed in British Patent No. 861,367; fenquizone, which may be prepared as disclosed in U.S. Patent No. 3,870,720; indapamide, which may be prepared as disclosed in U.S. Patent No. 3,565,911; hydrochlorothiazide, which may be prepared as disclosed in U.S. Patent No. 3,164,588; hydroflumethiazide, which may be prepared as disclosed in U.S. Patent No. 3,254,076; methyclothiazide, which may be prepared as disclosed in Close et al., Journal of the American Chemical Society, 1960, 82, 1132; meticrane, which may be prepared as disclosed in French Patent Nos. M2790 and 1,365,504; metolazone, which may be prepared as disclosed in U.S. Patent No. 3,360,518; paraflutizide, which may be prepared as disclosed in Belgian Patent No. 620,829; polythiazide, which may be prepared as disclosed in U.S. Patent No. 3,009,911; quinethazone, which may be prepared as disclosed in U.S. Patent No. 2,976,289; teclothiazide, which may be prepared as disclosed in Close et al., Journal of the American Chemical Society, 1960, 82, 1132; and trichlormethiazide, which may be prepared as disclosed in deStevens et al., Experientia, 1960, 16, 113.

Diuretic sulfonamide derivatives within the scope of this invention include acetazolamide, which may be prepared as disclosed in U.S. Patent No. 2,980,679; ambuside, which may be prepared as disclosed in U.S. Patent No. 3,188,329; azosemide, which may be prepared as disclosed in U.S. Patent No. 3,665,002; bumetanide, which may be prepared as disclosed in U.S. Patent No. 3,634,583; butazolamide, which may be prepared as disclosed in British Patent No. 769,757; chloraminophenamide, which may be prepared as disclosed in U.S. Patent Nos. 2,809,194, 2,965,655 and 2,965,656; clofenamide, which may be prepared as disclosed in Olivier, Rec. Trav. Chim., 1918, 37, 307; clopamide, which may be prepared as disclosed in U.S. Patent No. 3,459,756; clorexolone, which may be prepared as disclosed in U.S. Patent No. 3,183,243; disulfamide, which may be prepared as disclosed in British Patent No. 851,287; ethoxolamide, which may be prepared as disclosed in British Patent No. 795,174; furosemide, which may be prepared as disclosed in U.S. Patent No. 3,058,882; mefruside, which may be prepared as disclosed in U.S. Patent No. 3,356,692; methazolamide, which may be prepared as disclosed in U.S. Patent No. 2,783,241; piretanide, which may be prepared as disclosed in U.S. Patent No. 4,010,273; torasemide, which may be prepared as disclosed in U.S. Patent No. 4,018,929; tripamide, which may be prepared as disclosed in Japanese Patent No. 73 05,585; and xipamide, which may be prepared as disclosed in U.S. Patent No. 3,567,777.

In addition, atorvastatin and pharmaceutically acceptable salts thereof may occur as hydrates or solvates. Further, the alpha-adrenergic receptor blocker and diuretic agents which may be used in accordance with this invention and the pharmaceutically acceptable salts thereof may occur as hydrates or solvates. Said hydrates and solvates are also within the scope of the invention.

The pharmaceutical combinations and methods of this invention are all adapted to therapeutic use as agents in the treatment of atherosclerosis, angina pectoris, and a condition characterized by the presence of both hypertension and hypertipidemia in mammals, particularly humans. Further, since these diseases and conditions are closely related to the development of cardiac disease and adverse cardiac conditions, these combinations and methods, by virtue of their action as antiatheroscterotics, antianginals, antihypertensives and antihyperlipidemics, are useful in the management of cardiac risk.

The utility of the compounds of the present invention as medical agents in the treatment of atherosclerosis in mammals (e.g. humans) is demonstrated by the activity of the compounds of this invention in conventional assays and the clinical protocol described below:

### Effect of Atorvastatin and an Alpha-adrenergic Receptor Blocker or Diuretic, Alone and in Combination, on the Treatment of Atherosclerosis

This study is a prospective randomized evaluation of the effect of a combination of atorvastatin or a pharmaceutically acceptable salt thereof and an alpha-adrenergic receptor blocker or diuretic agent on the progression/regression of coronary and carotid artery disease. The study is used to show that a combination of atorvastatin or a pharmaceutically acceptable salt thereof and an alpha-adrenergic receptor blocker or diuretic agent is effective in slowing or arresting the progression or causing regression of existing coronary artery disease (CAD) as evidenced by changes in coronary angiography or carotid ultrasound, in subjects with established disease.

This study is an angiographic documentation of coronary artery disease carried out as a double-blind, placebo-controlled trial of a minimum of about 500 subjects and preferably of about 780 to about 1200 subjects. It is especially preferred to study about 1200 subjects in this study. Subjects are admitted into the study after satisfying certain entry criteria set forth below.

Entry criteria: Subjects accepted for entry into this trial must satisfy certain criteria. Thus the subject must be an adult, either male or female, aged 18-80 years of age in whom coronary angiography is clinically indicated. Subjects will have angiographic presence of a significant focal lesion such as 30% to 50% on subsequent evaluation by quantitative coronary angiography (QCA) in a minimum of one segment (non-PTCA, non-bypassed or non-MI vessel) that is judged not likely to require intervention over the next 3 years. It is required that the segments undergoing analysis have not been interfered with. Since percutaneous transluminal cardiac angioplasty (PTCA) interferes with segments by the insertion of a balloon catheter, non-PTCA segments are required for analysis. It is also required that the segments to be analyzed have not suffered a thrombotic event, such as a myocardial infarct (MI). Thus the requirement for non-MI vessels. Segments that will be analyzed include: left main, proximal, mid and distal left anterior descending, first and second diagonal branch, proximal and distal left circumflex, first or largest space obtuse marginal, proximal, mid and distal right coronary artery. Subjects will have an ejection fraction of greater than 40% determined by catheterization or radionuclide ventriculography or ECHO cardiogram at the time of the qualifying angiogram or within the previous three months of the acceptance of the qualifying angiogram provided no intervening event such as a thrombotic event or procedure such as PTCA has occurred.

Generally, due to the number of patients and the physical limitations of any one facility, the study is carried out at multiple sites. At entry into the study, subjects undergo quantitative coronary angiography as well as B-mode carotid artery ultrasonography and assessment of carotid arterial compliance at designated testing centers. This establishes baselines for each subject. Once admitted into the test, subjects are randomized to receive an alpha-adrenergic receptor blocker or diuretic agent or a pharmaceutically acceptable salt thereof ( the dose is dependent upon the particular alpha-adrenergic receptor blocker or diuretic agent or salt thereof chosen) and placebo or atorvastatin calcium (80 mgs) and placebo or an alpha-adrenergic receptor blocker or diuretic agent or a pharmaceutically acceptable salt thereof (the dose is dependent upon the particular alpha-adrenergic receptor blocker or diuretic agent or salt thereof chosen) and atorvastatin calcium (80 mgs). It will be recognized by a skilled person that the free base form or other salt forms of the alpha-adrenergic receptor blocker or diuretic, or the free base form or other salt forms of the statin may be used in this invention. Calculation of the dosage amount for these other forms of the statin and other agent is easily accomplished by performing a simple ratio relative to the molecular weights of the species involved. The amount of the alpha-adrenergic receptor blocker or diuretic agent may be varied as required. The amount of the statin will be titrated down from 80 mg if it is determined by the physician to be in the best interests of the subject. The subjects are monitored for a one to three year period, generally three years being preferred. B-mode carotid ultrasound assessment of carotid artery atherosclerosis and compliance are performed at regular intervals throughout the study.

Generally, six month intervals are suitable. Typically this assessment is performed using B-mode ultrasound equipment. However, a person skilled in the art may use other methods of performing this assessment. Coronary angiography is performed at the conclusion of the one to three year treatment period. The baseline and post-treatment angiograms and the intervening carotid artery B-mode ultrasonograms are evaluated for new lesions or progression of existing atherosclerotic lesions. Arterial compliance measurements are assessed for changes from baseline and over the 6-month evaluation periods.

The primary objective of this study is to show that the combination of an alpha-adrenergic receptor blocker or diuretic agent and atorvastatin reduces the progression of atherosclerotic lesions as measured by quantitative coronary angiography (QCA) in subjects with clinical coronary artery disease. QCA measures the opening in the lumen of the arteries measured.

The primary endpoint of the study is the change in the average mean segment diameter of the coronary artery tree. Thus, the diameter of an arterial segment is measured at various portions along the length of that segment. The average diameter of that segment is then determined. After the average segment diameter of many segments has been determined, the average of all segment averages is determined to arrive at the average mean segment diameter. The mean segment diameter of subjects taking atorvastatin or a pharmaceutically acceptable salt thereof and an alpha-adrenergic receptor blocker or diuretic agent or a pharmaceutically acceptable acid addition salt thereof will decline more slowly, will be halted completely, or there will be an increase in the mean segment diameter. These results represent slowed progression of atherosclerosis, halted progression of atherosclerosis and regression of atherosclerosis, respectively.

The secondary objective of this study is that the combination of an alpha-adrenergic receptor blocker or diuretic agent and atorvastatin or a pharmaceutically acceptable salt thereof reduces the rate of progression of atherosclerosis in the carotid arteries as measured by the slope of the maximum intimal-medial thickness measurements averaged over 12 separate wall segments (Mean Max) as a function of time, more than does amlodipine or a pharmaceutically acceptable acid addition salt thereof or atorvastatin or a pharmaceutically acceptable salt thereof alone. The intimal-medial thickness of subjects taking atorvastatin or a pharmaceutically acceptable salt thereof and an alpha-adrenergic receptor blocker or diuretic agent or a pharmaceutically acceptable acid addition salt thereof will increase more slowly, will cease to increase or will decrease. These results represent slowed progression of atherosclerosis, hafted progression of atherosclerosis and regression of atherosclerosis, respectively. Further, these results may be used to facilitate dosage determinations.

The utility of the compounds of the present invention as medical agents in the treatment of angina pectoris in mammals (e.g., humans) is demonstrated by the activity of the compounds of this invention in conventional assays and the clinical protocol described below:

### Effect of Atorvastatin and an Alpha-adrenergic Receptor Blocker or Diuretic Agent, Alone and in Combination, on the Treatment of Angina

This study is a double blind, parallel arm, randomized study to show the effectiveness of atorvastatin or a pharmaceutically acceptable salt thereof and an alpha-adrenergic receptor blocker or diuretic agent given in combination in the treatment of symptomatic angina.

Entry criteria: Subjects are males or females between 18 and 80 years of age with a history of typical chest pain associated with one of the following objective evidences of cardiac ischemia: (1) stress test segment elevation of about one millimeter or more from the ECG; (2) positive treadmill stress test; (3) new wall motion abnormality on ultrasound; or (4) coronary angiogram with a significant qualifying stenosis. Generally a stenosis of about 30-50% is considered to be significant.

Each subject is evaluated for about ten to thirty-two weeks. At least ten weeks are generally required to complete the study. Sufficient subjects are used in this screen to ensure that about 200 to 800 subjects and preferably about 400 subject are evaluated to complete the study. Subjects are screened for compliance with the entry criteria, set forth below, during a four week run in phase. After the screening criteria are met, subjects are washed out from their current anti-anginal medication and stabilized on a long acting nitrate such as nitroglycerine, isosorbide-5-mononitrate or isosorbide dinitrate. The term "washed out", when used in connection with this screen, means the withdrawal of current anti-anginal medication so that substantially all of said medication is eliminated from the body of the subject. A period of eight weeks is preferably allowed for both the wash out period and for the establishment of the subject on stable doses of said nitrate. Subjects having one or two attacks of angina per week while on stable doses of long acting nitrate are generally permitted to skip the wash out phase. After subjects are stabilized on nitrates, the subjects enter the randomization phase provided the subjects continue to have either one or two angina attacks per week. In the randomization phase, the subjects are randomly placed into one of the four arms of the study set forth below. After completing the wash out phase, subjects in compliance with the entry criteria undergo twenty four hour ambulatory electrocardigram (ECG) such as Holter monitoring, exercise stress testing such as a treadmill and evaluation of myocardial perfusion using PET (photon emission tomography) scanning to establish a baseline for each subject. When conducting a stress test, the speed of the treadmill and the gradient of the treadmill can be controlled by a technician. The speed of the treadmill and the angle of the gradient are generally increased during the test. The time intervals between each speed and gradient increase is generally determined using a modified Bruce Protocol.

After the baseline investigations have been completed, subjects are initiated on one of the following four arms of the study: (1) placebo; (2) atorvastatin (about 10 mg to about 80 mg); (3) an alpha-adrenergic receptor blocker or diuretic (dose is dependent upon the particular agent chosen); or (4) a combination of the above doses of atorvastatin and agent together. It will be recognized by a skilled person that the free base form or other salt forms of the alpha-adrenergic receptor blocker or diuretic or the free base form or other salt forms of the statin may be used in this invention. Calculation of the dosage amount for these other forms of the statin and alpha-adrenergic receptor blocker or diuretic is easily accomplished by performing a simple ratio relative to the molecular weights of the species involved. The subjects are then monitored for two to twenty four weeks.

After the monitoring period has ended, subjects will undergo the following investigations: (1) twenty four hour ambulatory ECG, such as Holter monitoring; (2) exercise stress testing (e.g. treadmill using said modified Bruce Protocol); and (3) evaluation of myocardial perfusion using PET scanning. Patients keep a diary of painful ischemic events and nitroglycerine consumption. It is generally desirable to have an accurate record of the number of anginal attacks suffered by the patient during the duration of the test. Since a patient generally takes nitroglycerin to ease the pain of an anginal attack, the number of times that the patient administers nitroglycerine provides a reasonably accurate record of the number of anginal attacks.

To demonstrate the effectiveness and dosage of the drug combination of this invention, the person conducting the test will evaluate the subject using the tests described. Successful treatment will yield fewer instances of ischemic events as detected by ECG, will allow the subject to exercise longer or at a higher intensity level on the treadmill, or to exercise without pain on the treadmill, or will yield better perfusion or fewer perfusion defects on ultrasound.

The utility of the compounds of the present invention as medical agents in the treatment of hypertension and hyperlipidemia in mammals (e.g., humans) suffering from a combination of hypertension and hypertipidemia is demonstrated by the activity of the compounds of this invention in conventional assays and the clinical protocol described below:

### Effect of Atorvastatin and an Alpha-adrenergic Receptor Blocker or Diuretic Agent, Alone and in Combination, on the Treatment of Subjects Having Both Hypertension and Hyperlipidemia

This study is a double blind, parallel arm, randomized study to show the effectiveness of atorvastatin or a pharmaceutically acceptable salt thereof and an alpha-adrenergic receptor blocker or diuretic agent given in combination in controlling both hypertension and hyperlipidemia in subjects who have mild, moderate, or severe hypertension and hyperlipidemia.

Each subject is evaluated for 10 to 20 weeks and preferably for 14 weeks. Sufficient subjects are used in this screen to ensure that about 400 to 800 subjects are evaluated to complete the study.

Entry criteria: Subjects are male or female adults between 18 and 80 years of age having both hyperlipidemia and hypertension. The presence of hyperlipidemia is evidenced by evaluation of the low density lipoprotein (LDL) level of the subject relative to certain positive risk factors. If the subject has no coronary heart disease (CHD) and has less than two positive risk factors, then the subject is considered to have hyperlipidemia which requires drug therapy if the LDL of the subject is greater than or equal to 190. If the subject has no CHD and has two or more positive risk factors, then the subject is considered to have hyperlipidemia which requires drug therapy if the LDL of the subject is greater than or equal to 160. If the subject has CHD, then the subject is considered to have hypertipidemia if the LDL of the subject is greater than or equal to 130.

Positive risk factors include (1) male over 45, (2) female over 55 wherein said female is not undergoing hormone replacement therapy (HRT), (3) family history of premature cardiovascular disease, (4) the subject is a current smoker, (5) the subject has diabetes, (6) an HDL of less than 45, and (7) the subject has hypertension. An HDL of greater than 60 is considered a negative risk factor and will offset one of the above mentioned positive risk factors.

The presence of hypertension is evidenced by a sitting diastolic blood pressure (BP) of greater than 90 or sitting systolic BP of greater than 140. All blood pressures are generally determined as the average of three measurements taken five minutes apart.

Subjects are screened for compliance with the entry criteria set forth above. After all screening criteria are met, subjects are washed out from their current antihypertensive and lipid lowering medication and are placed on the NCEP ATP II Step 1 diet. The NCEP ATP II (adult treatment panel, 2nd revision) Step 1 diet sets forth the amount of saturated and unsaturated fat which can be consumed as a proportion of the total caloric intake. The term "washed out" where used in connection with this screen, means the withdrawal of current antihypertensive and lipid lowering medication so that substantially all of said medication is eliminated from the body of the subject. Newly diagnosed subjects generally remain untreated until the test begins. These subjects are also placed on the NCEP Step 1 diet. After the four week wash out and diet stabilization period, subjects undergo the following baseline investigations: (1) blood pressure and (2) fasting lipid screen. The fasting lipid screen determines baseline lipid levels in the fasting state of a subject. Generally, the subject abstains from food for twelve hours, at which time lipid levels are measured.

After the baseline investigations are performed subjects are started on one of the following: (1) a fixed dose of an alpha-adrenergic receptor blocker or diuretic agent, dose dependent upon the particular agent chosen; (2) a fixed dose of atorvastatin, generally about 10 to 80mg; or (3) a combination of the above doses of atorvastatin and an alpha-adrenergic receptor blocker or diuretic agent together. It will be recognized by a skilled person that the free base form or other salt forms of an alpha-adrenergic receptor blocker or diuretic agent or the free base form or other salt forms of the statin may be used in this invention. Calculation of the dosage amount for these other forms of the statin and alpha-adrenergic receptor blocker or diuretic agent is easily accomplished by performing a simple ratio relative to the molecular weights of the species involved. Subjects remain on these doses for a minimum of six weeks, and generally for no more than eight weeks. The subjects return to the testing center at the conclusion of the six to eight weeks so that the baseline evaluations can be repeated. The blood pressure of the subject at the conclusion of the study is compared with the blood pressure of the subject upon entry. The lipid screen measures the total cholesterol, LDL-cholesterol, HDL-cholesterol, triglycerides, apoB, VLDL (very low density lipoprotein) and other components of the lipid profile of the subject. Improvements in the values obtained after treatment relative to pretreatment values indicate the utility of the drug combination.

The utility of the compounds of the present invention as medical agents in the management of cardiac risk in mammals (e.g., humans) at risk for an adverse cardiac event is demonstrated by the activity of the compounds of this invention in conventional assays and the clinical protocol described below:

### Effects of Atorvastatin and an Alpha-adrenergic Receptor Blocker or Diuretic Agent, Alone and in Combination, on Subjects at Risk of Future Cardiovascular Events

This study is a double blind, parallel arm, randomized study to show the effectiveness of atorvastatin or a pharmaceutically acceptable salt thereof and an alpha-adrenergic receptor blocker or diuretic agent given in combination in reducing the overall calculated risk of future events in subjects who are at risk for having future cardiovascular events. This risk is calculated by using the Framingham Risk Equation. A subject is considered to be at risk of having a future cardiovascular event if that subject is more than one standard deviation above the mean as calculated by the Framingham Risk Equation. The study is used to evaluate the efficacy of a fixed combination of atorvastatin or a pharmaceutically acceptable salt thereof and an antihypertensive agent in controlling cardiovascular risk by controlling both hypertension and hyperlipidemia in patients who have both mild to moderate hypertension and hyperlipidemia.

Each subject is evaluated for 10 to 20 weeks and preferably for 14 weeks. Sufficient subjects are recruited to ensure that about 400 to 800 subjects are evaluated to complete the study.

Entry criteria: Subjects included in the study are male or female adult subjects between 18 and 80 years of age with a baseline five year risk which risk is above the median for said subject's age and sex, as defined by the Framingham Heart Study, which is an ongoing prospective study of adult men and women showing that certain risk factors can be used to predict the development of coronary heart disease. The age, sex, systolic and diastolic blood pressure, smoking habit, presence or absence of carbohydrate iritolerance, presence or absence of left ventricular hypertrophy, serum cholesterol and high density lipoprotein (HDL) of more than one standard deviation above the norm for the Framingham Population are all evaluated in determining whether a patient is at risk for adverse cardiac event. The values for the risk factors are inserted into the Framingham Risk equation and calculated to determine whether a subject is at risk for a future cardiovascular event.

Subjects are screened for compliance with the entry criteria set forth above. After all screening criteria are met, patients are washed out from their current antihypertensive and lipid lowering medication and any other medication which will impact the results of the screen. The patients are then placed on the NCEP ATP II Step 1 diet, as described above. Newly diagnosed subjects generally remain untreated until the test begins. These subjects are also placed on the NCEP ATP II Step 1 diet. After the four week wash out and diet stabilization period, subjects undergo the following baseline investigations: (1) blood pressure; (2) fasting; (3) lipid screen; (4) glucose tolerance test; (5) ECG; and (6) cardiac ultrasound. These tests are carried out using standard procedures well known to persons skilled in the art. The ECG and the cardiac ultrasound are generally used to measure the presence or absence of left ventricular hypertrophy.

After the baseline investigations are performed patients will be started on one of the following: (1) a fixed dose of an alpha-adrenergic receptor blocker or diuretic agent, dose dependent upon the particular agent chosen; (2) a fixed dose of atorvastatin (about 10 to 80mg); or (3) the combination of the above doses of atorvastatin and an alpha-adrenergic receptor blocker or diuretic agent. It will be recognized by a skilled person that the free base form or other salt forms of the alpha-adrenergic receptor blocker or diuretic agent or the free base form or other salt forms of the statin may be used in this invention. Calculation of the dosage amount for these other forms of the statin and alpha-adrenergic or diuretic agent is easily accomplished by performing a simple ratio relative to the molecular weights of the species involved. Patients are kept on these doses and are asked to return in six to eight weeks so that the baseline evaluations can be repeated. At this time the new values are entered into the Framingham Risk equation to determine whether the subject has a lower, greater or no change in the risk of future cardiovascular event.

The above assays demonstrating the effectiveness of an alpha-adrenergic or diuretic agent or pharmaceutically acceptable acid addition salts thereof and atorvastatin or pharmaceutically acceptable salts thereof in the treatment of angina pectoris, atherosclerosis, hypertension and hypertipidemia together, and the management of cardiac risk, also provide a means whereby the activities of the compounds of this invention can be compared between themselves and with the activities of other known compounds. The results of these comparisons are useful for determining dosage levels in mammals, including humans, for the treatment of such diseases.

The following dosage amounts and other dosage amounts set forth elsewhere in this specification and in the appendant claims are for an average human subject having a weight of about 65 kg to about 70 kg. The skilled practitioner will readily be able to determine the dosage amount required for a subject whose weight falls outside the 65 kg to 70 kg range, based upon the medical history of the subject and the presence of diseases, e.g., diabetes, in the subject. All doses set forth herein, and in the appendant claims, are daily doses.

In general, in accordance with this invention, the below-listed alpha-adrenergic receptor blocker or diuretic agent is to be administered in the following dosage amounts:
doxazosin, generally about 0.5 mg to about 16 mg;
prazosin, generally about 1 mg to about 40 mg;
trimazosin, generally about 1 mg to about 20 mg; and
amiloride, generally about 5 mg to about 20 mg.

It will be recognized by those skilled in the art that dosages for the above compounds must be individualized to each specific subject. This individualization will depend upon the medical history of the subject and whether the subject is concurrently taking other medications which may or may not interfere or have an adverse effect in combination with the above compounds. Individualization is then achieved by beginning with a low dose of the compound and titrating the amount up until the desired therapeutic effect is achieved.

In general, in accordance with this invention, atorvastatin calcium is generally to be administered in a dosage of about 2.5 mg to about 160 mg. Preferably, atorvastatin calcium is administered in a dosage of about 10 mg to about 80 mg.

The compounds of the present invention are generally to be administered in the form of a pharmaceutical composition comprising at least one of the compounds of this invention together with a pharmaceutically acceptable carrier or diluent. Thus, the compounds of this invention can be administered either individually or together in any conventional oral, parenteral or transdermal dosage form.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

The combination of this invention may also be administered in a controlled-release dosage formulation such as a slow release or a fast release formulation. Such controlled release dosage formulations of the combination of this invention may be prepared according to methods well known to those skilled in the art. The method of administration will be determined by the attendant physician after an evaluation of the subject's condition and requirements.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

Methods of preparing various pharmaceutical compositions with a certain amount of active ingredient are known, or will be apparent in light of this disclosure, to those skilled in this art. For examples, see Remington's Pharmaceutical Sciences, Mack Publishing Company, Easter, Pa., 15th Edition (1975).

Pharmaceutical compositions according to the invention may contain 0.1%-95% of the compound(s) of this invention, preferably 1%-70%. In any event, the composition or formulation to be administered will contain a quantity of a compound(s) according to the invention in an amount effective to treat the condition or disease of the subject being treated.

Since the present invention relates to the use in the treatment of diseases and conditions with a combination of active ingredients which may be administered separately, the invention also relates to combining separate pharmaceutical compositions in kit form. The kit includes two separate pharmaceutical compositions: an alpha-adrenergic receptor blocker or diuretic agent or a pharmaceutically acceptable salt thereof, and atorvastatin or a pharmaceutically acceptable salt thereof. The kit includes container means for containing the separate compositions such as a divided bottle or a divided foil packet. However, the separate compositions may also be contained within a single, undivided container. Typically the kit includes directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

## Claims

1. A pharmaceutical composition comprising:
a. an amount of atorvastatin or a pharmaceutically acceptable salt thereof;
b. an amount of an alpha-adrenergic receptor blocker or a pharmaceutically acceptable salt thereof; and
c. a pharmaceutically acceptable carrier or diluent

2. A pharmaceutical product comprising
a. an amount of an alpha-adrenergic receptor blocker or a pharmaceutically acceptable salt thereof and a pharmaceutically acceptable carrier or diluent;
b. an amount of atorvastatin or a pharmaceutically acceptable salt thereof, and a pharmaceutically acceptable carrier or diluent;
as a combined preparation for simultaneous or sequential use in treating angina pectoris, atherosclerosis, combined hypertension and hyperlipidemia and use in cardiac risk management.

3. A composition or product according to claim 1 or claim 2, comprising the hemicalcium salt of atorvastatin.

4. A composition or product according to any preceding claim, wherein the alpha-adrenergic receptor blocker is amosulalol, arotinolol, dapiprazole, doxazosin, fenspiride, indoramin, labetolol, naftopidil, nicergoline, prazosin, tamsulosin, totazoline, trimazosin, yohimbine or a pharmaceutically acceptable salt thereof.

5. A composition or product according to claim 4, wherein the alpha-adrenergic receptor blocker is doxazosin, prazosin, trimazosin or a pharmaceutically acceptable salt thereof.

6. Use of
(a) a first compound, said first compound being atorvastatin or a pharmaceutically acceptable salt thereof; and
(b) a second compound, said second compound being an alpha adrenergic receptor blocker or a pharmaceutically acceptable salt thereof;
in the preparation of a medicament for the treatment of angina pectoris, atherosclerosis, combined hypertension and hyperlipidemia and in cardiac risk management.

7. Use according to claim 6, wherein said first and second compounds are as further defined in any one of claims 3 to 5.

8. A kit for achieving a therapeutic effect in a mammal comprising:
a. an amount of atorvastatin or a pharmaceutically acceptable salt thereof and a pharmaceutically acceptable carrier or diluent in a first unit dosage form;
b. an amount of an alpha-adrenergic receptor blocker or a pharmaceutically acceptable salt thereof and a pharmaceutically acceptable carrier or diluent in a second unit dosage form; and
c. container means for containing said first and second dosage forms.

9. A kit according to claim 8, wherein the atorvastatin and alpha-adrenergic receptor blocker are as further defined in claims 3 to 5.

10. Use of a kit according to claims 8 and 9, for the preparation of a medicament for the treatment of angina pectoris, atherosclerosis, combined hypertension and hyperlipidemia and in cardiac risk management.

11. Use according to claim 10, wherein said first and second compounds are in separate dosage forms and are adapted for simultaneous or sequential administration.

12. A pharmaceutical composition comprising:
a. an amount of atorvastatin or a pharmaceutically acceptable salt thereof;
b. an amount of a diuretic or a pharmaceutically acceptable salt thereof; and
c. a pharmaceutically acceptable carrier or diluent

13. A pharmaceutical product comprising
a. an amount of a diuretic or a pharmaceutically acceptable salt thereof and a pharmaceutically acceptable carrier or diluent;
b. an amount of atorvastatin or a pharmaceutically acceptable salt thereof, and a pharmaceutically acceptable carrier or diluent;
as a combined preparation for simultaneous or sequential use in treating angina pectoris, atherosclerosis, combined hypertension and hyperlipidemia and use in cardiac risk management.

14. A composition or a product according to claims 12 and 13, comprising the hemicalcium salt of atorvastatin.

15. A composition or product according to any of claims 12 to 14, wherein the diuretic is a benzothiadiazine derivative, organomercurial, purine, steroid, sulfonamide derivative, uracil or a pharmaceutically acceptable salt thereof.

16. A composition or product according to claim 15, wherein the diuretic is amanozine, amiloride, arbutin, chlorazanil, ethacrynic acid, etozolin, hydracarbazine, isosorbide, mannitol, metochalcone, muzolimine, perhexiline, ticrynafen, triamterene, urea or a pharmaceutically acceptable salt thereof.

17. A composition or product according to claim 15, wherein the diuretic is amiloride or bendroflumethiazide.

18. Use of
(a) a first compound, said first compound being atorvastatin or a pharmaceutically acceptable salt thereof; and
(b) a second compound, said second compound being a diuretic or a pharmaceutically acceptable salt thereof;
in the preparation of a medicament for the treatment of angina pectoris, atherosclerosis, combined hypertension and hyperlipidemia and in cardiac risk management.

19. Use according to claim 18, wherein said first and second compounds are as further defined in any one of claims 14 to 17.

20. A kit for achieving a therapeutic effect in a mammal comprising:
a. an amount of atorvastatin or a pharmaceutically acceptable salt thereof and a pharmaceutically acceptable carrier or diluent in a first unit dosage form;
b. an amount of a diuretic or a pharmaceutically acceptable salt thereof and a pharmaceutically acceptable carrier or diluent in a second unit dosage form; and
c. container means for containing said first and second dosage forms.

21. A kit according to claim 20, wherein the atorvastatin, therapeutic effect and diruetic are as further defined in claims 14 to 17.

22. Use of a kit according to claims 20 and 21, for the preparation of a medicament for the treatment of angina pectoris, atherosclerosis, combined hypertension and hyperlipidemia and in cardiac risk management.

23. Use according to claim 22, wherein said first and second compounds are in separate dosage forms and are adapted for simultaneous or sequential administration.

## Patentansprüche

1. Pharmazeutische Zusammensetzung, enthaltend:
a. eine Menge von Atorvastatin oder eines pharmazeutisch annehmbaren Salzes davon;
b. eine Menge eines alfa-adrenergen Rezeptorblockers oder eines pharmazeutisch annehmbaren Salzes davon; und
c. ein(en) pharmazeutisch annehmbaren(s) Träger oder Verdünnungsmittel.

2. Pharmazeutisches Produkt, enthaltend:
a. eine Menge eines alfa-adrenergen Rezeptorblockers oder eines pharmazeutisch annehmbaren Salzes davon und ein(en) pharmazeutisch annehmbaren(s) Träger oder Verdünnungsmittel;
b. eine Menge von Atorvastatin oder eines pharmazeutisch annehmbaren Salzes davon, und ein(en) pharmazeutisch annehmbaren(s) Träger oder Verdünnungsmittel;
als eine kombinierte Zubereitung zur simultanen oder sequenziellen Verwendung zur Behandlung von Angina pectoris, Atherosklerose, kombinierter Hypertonie und Hyperlipidämie und zur Verwendung für das kardiale Risikomanagement.

3. Zusammensetzung oder Produkt nach Anspruch 1 oder 2, enthaltend das Hemikalziumsalz von Atorvastatin.

4. Zusammensetzung oder Produkt nach irgend einem der vorangehenden Ansprüche, wobei der alfa-adrenerge Rezeptorblocker Amosulalol, Arotinolol, Dapiprazol, Doxazosin, Fenspirid, Indoramin, Labetolol, Naftopidil, Nicergolin, Prazosin, Tamsulosin, Tolazolin, Trimazosin, Yohimbin oder ein pharmazeutisch annehmbares Salz davon ist.

5. Zusammensetzung oder Produkt nach Anspruch 4, wobei der alfa-adrenerge Rezeptorblocker Doxazosin, Prazosin, Trimazosin oder ein pharmazeutisch annehmbares Salz davon ist.

6. Verwendung von
(a) einer ersten Verbindung, wobei besagte erste Verbindung Atorvastatin oder ein pharmazeutisch annehmbares Salz davon ist; und
(b) einer zweiten Verbindung, wobei besagte zweite Verbindung ein alfa-adrenerger Rezeptorblocker oder ein pharmazeutisch annehmbares Salz davon ist;
zur Herstellung eines Medikamentes zur Behandlung von Angina pectoris, Atherosklerose, kombinierter Hypertonie und Hyperlipidämie und für das kardiale Risikomanagement.

7. Verwendung nach Anspruch 6, wobei besagte erste und zweite Verbindungen wie in einem der Ansprüche 3 bis 5 definiert sind.

8. Kit zum Bewirken eines therapeutischen Effektes in einem Säuger, enthaltend:
a. eine Menge von Atorvastatin oder eines pharmazeutisch annehmbaren Salzes davon und ein(en) pharmazeutisch annehmbaren(s) Träger oder Verdünnungsmittel in einer ersten Einheitsdosierungsform;
b. eine Menge eines alfa-adrenergen Rezeptorblockers oder eines pharmazeutisch annehmbaren Salzes davon und ein(en) pharmazeutisch annehmbaren(s) Träger oder Verdünnungsmittel in einer zweiten Einheitsdosierungsform; und
c. Behältnismittel zur Aufnahme von besagten ersten und zweiten Einheitsdosierungsformen.

9. Kit nach Anspruch 8, wobei das Atorvastatin und der alfa-adrenerge Rezeptorblocker wie in den Ansprüchen 3 bis 5 definiert sind.

10. Verwendung eines Kits nach den Ansprüchen 8 und 9 zur Herstellung eines Medikamentes zur Behandlung von Angina pectoris, Atherosklerose, kombinierter Hypertonie und Hyperlipidämie und für das kardiale Risikomanagement.

11. Verwendung nach Anspruch 10, wobei besagte erste und zweite Verbindungen in getrennten Dosierungsformen vorliegen und zur simultanen oder sequenziellen Verabreichung angepasst sind.

12. Pharmazeutische Zusammensetzung, enthaltend:
a. eine Menge von Atorvastatin oder eines pharmazeutisch annehmbaren Salzes davon;
b. eine Menge eines Diuretikums oder eines pharmazeutisch annehmbaren Salzes davon; und
c. ein(en) pharmazeutisch annehmbaren(s) Träger oder Verdünnungsmittel.

13. Pharmazeutisches Produkt, enthaltend:
a. eine Menge eines Diuretikums oder eines pharmazeutisch annehmbaren Salzes davon und ein(en) pharmazeutisch annehmbaren(s) Träger oder Verdünnungsmittel;
b. eine Menge von Atorvastatin oder eines pharmazeutisch annehmbaren Salzes davon, und ein(en) pharmazeutisch annehmbaren(s) Träger oder Verdünnungsmittel; als eine kombinierte Zubereitung zur simultanen oder sequenziellen Verwendung zur Behandlung von Angina pectoris, Atherosklerose, kombinierter Hypertonie und Hyperlipidämie und zur Verwendung für das kardiale Risikomanagement.

14. Zusammensetzung oder Produkt nach den Ansprüchen 12 und 13, enthaltend das Hemikalziumsalz von Atorvastatin.

15. Zusammensetzung oder Produkt nach irgend einem der Ansprüche 12 bis 14, wobei das Diuretikum ein Benzothiadiazin-Derivat, ein Organoquecksilber, ein Purin, ein Steroid, ein Sulfonamid-Derivat, Uracil oder ein pharmazeutisch annehmbares Salz davon ist.

16. Zusammensetzung oder Produkt nach Anspruch 15, wobei das Diuretikum Amanozin, Amilorid, Arbutin, Chlorazanil, Ethacrynsäure, Etozolin, Hydracarbazin, Isosorbid, Mannitol, Metochalcon, Muzolimin, Perhexilin, Ticrynafen, Triamteren, Harnstoff oder ein pharmazeutisch annehmbares Salz davon ist.

17. Zusammensetzung oder Produkt nach Anspruch 15, wobei das Diuretikum Amilorid oder Bendroflumethiazid ist.

18. Verwendung von
(a) einer ersten Verbindung, wobei besagte erste Verbindung Atorvastatin oder ein pharmazeutisch annehmbares Salz davon ist; und
(b) einer zweiten Verbindung, wobei besagte zweite Verbindung ein Diuretikum oder ein pharmazeutisch annehmbares Salz davon ist; zur Herstellung eines Medikamentes zur Behandlung von Angina pectoris, Atherosklerose, kombinierter Hypertonie und Hyperlipidämie und für das kardiale Risikomanagement.

19. Verwendung nach Anspruch 18, wobei besagte erste und zweite Verbindungen wie in einem der Ansprüche 14 bis 17 definiert sind.

20. Kit zum Bewirken eines therapeutischen Effektes in einem Säuger, enthaltend:
a. eine Menge von Atorvastatin oder eines pharmazeutisch annehmbaren Salzes davon und ein(en) pharmazeutisch annehmbaren(s) Träger oder Verdünnungsmittel in einer ersten Einheitsdosierungsform;
b. eine Menge eines Diuretikums oder eines pharmazeutisch annehmbaren Salzes davon und ein(en) pharmazeutisch annehmbaren(s) Träger oder Verdünnungsmittel in einer zweiten Einheitsdosierungsform; und
c. Behältnismittel zur Aufnahme von besagten ersten und zweiten Einheitsdosierungsformen.

21. Kit nach Anspruch 20, wobei das Atorvastatin, die therapeutische Wirkung und das Diuretikum wie in den Ansprüchen 14 bis 17 definiert sind.

22. Verwendung eines Kits nach den Ansprüchen 20 und 21 zur Herstellung eines Medikamentes zur Behandlung von Angina pectoris, Atherosklerose, kombinierter Hypertonie und Hyperlipidämie und für das kardiale Risikomanagement.

23. Verwendung nach Anspruch 22, wobei besagte erste und zweite Verbindungen in getrennten Dosierungsformen vorliegen und zur simultanen oder sequenziellen Verabreichung angepasst sind.

## Revendications

1. Composition pharmaceutique comprenant :
a. une quantité d'atorvastatine ou d'un de ses sels pharmaceutiquement acceptables ;
b. une quantité d'un agent de blocage des récepteurs alpha-adrénergiques ou d'un de ses sels pharmaceutiquement acceptables ; et
c. un support ou diluant pharmaceutiquement acceptable.

2. Produit pharmaceutique comprenant :
a. une quantité d'un agent de blocage des récepteurs alpha-adrénergiques ou d'un de ses sels pharmaceutiquement acceptables, et un support ou diluant pharmaceutiquement acceptable ;
b. une quantité d'atorvastatine ou d'un de ses sels pharmaceutiquement acceptables, et un support ou diluant pharmaceutiquement acceptable ;
sous forme d'une préparation combinée pour une utilisation simultanée ou séquentielle dans le traitement de l'angine de poitrine, de l'athérosclérose, de l'hypertension et de l'hyperlipidémie combinées et pour une utilisation dans la prise en compte du risque cardiaque.

3. Composition ou produit suivant la revendication 1 ou la revendication 2, comprenant le sel hémicalcique d'atorvastatine.

4. Composition ou produit suivant l'une quelconque des revendications précédentes, dans laquelle l'agent de blocage des récepteurs alpha-adrénergiques est l'amosulalol, l'arotinolol, le dapiprazole, la doxazosine, le fenspiride, l'indoramine, le labétolol, le naftopidil, la nicergoline, la prazosine, la tamsulosine, la tolazoline, la trimazosine, la yohimbine ou un de ses sels pharmaceutiquement acceptables.

5. Composition ou produit suivant la revendication 4, dans laquelle l'agent de blocage des récepteurs alpha-adrénergiques est la doxazosine, la prazosine, la trimazosine, ou un de ses sels pharmaceutiquement acceptables.

6. Utilisation
(a) d'un premier composé, ledit premier composé étant l'atorvastatine ou un de ses sels pharmaceutiquement acceptables ; et
(b) d'un second composé, ledit second composé étant un agent de blocage des récepteurs alpha-adrénergiques ou un de ses sels pharmaceutiquement acceptables ;
dans la préparation d'un médicament destiné au traitement de l'angine de poitrine, de l'athérosclérose, de l'hypertension et de l'hyperlipidémie combinées et dans la prise en compte du risque cardiaque.

7. Utilisation suivant la revendication 6, dans laquelle lesdits premier et second composés sont en outre définis dans l'une quelconque des revendications 3 à 5.

8. Kit pour obtenir un effet thérapeutique chez un mammifère, comprenant :
a. une quantité d'atorvastatine ou d'un de ses sels pharmaceutiquement acceptables et un support ou diluant pharmaceutiquement acceptable dans une première forme posologique unitaire ;
b. une quantité d'un agent de blocage des récepteurs alpha-adrénergiques ou d'un de ses sels pharmaceutiquement acceptables et un support ou diluant pharmaceutiquement acceptable dans une seconde forme posologique unitaire ; et
c. un moyen servant de récipient pour contenir lesdites première et seconde formes posologiques.

9. Kit suivant la revendication 8, dans lequel l'atorvastatine et l'agent de blocage des récepteurs alpha-adrénergiques sont en outre définis dans les revendications 3 à 5.

10. Utilisation d'un kit suivant les revendications 8 et 9, pour la préparation d'un médicament destiné au traitement de l'angine de poitrine, de l'athérosclérose, de l'hypertension et de l'hyperlipidémie combinées et dans la prise en compte du risque cardiaque.

11. Utilisation suivant la revendication 10, dans laquelle lesdits premier et second composés sont présents dans des formes posologiques distinctes et sont adaptés à l'administration simultanée ou séquentielle.

12. Composition pharmaceutique comprenant :
a. une quantité d'atorvastatine ou d'un de ses sels pharmaceutiquement acceptables ;
b. une quantité d'un diurétique ou d'un de ses sels pharmaceutiquement acceptables ; et
c. un support ou diluant pharmaceutiquement acceptable.

13. Produit pharmaceutique comprenant :
a. une quantité d'un diurétique ou d'un de ses sels pharmaceutiquement acceptables, et un support ou diluant pharmaceutiquement acceptable ;
b. une quantité d'atorvastatine ou d'un de ses sels pharmaceutiquement acceptables, et un support ou diluant pharmaceutiquement acceptable ;
sous forme d'une préparation combinée pour une utilisation simultanée ou séquentielle dans le traitement de l'angine de poitrine, de l'athérosclérose, de l'hypertension et de l'hyperlipidémie combinées et pour une utilisation dans la prise en compte du risque cardiaque.

14. Composition ou produit suivant les revendications 12 et 13, comprenant le sel hémicalcique d'atorvastatine.

15. Composition ou produit suivant l'une quelconque des revendications 12 à 14, dans laquelle le diurétique est un dérivé de benzothiadiazine, un composé organomercuriel, une purine, un stéroïde, un dérivé de sulfonamide, l'uracile ou un de ses sels pharmaceutiquement acceptables.

16. Composition ou produit suivant la revendication 15, dans laquelle le diurétique est l'amanozine, l'amiloride, l'arbutine, le chlorazanil, l'acide éthacrynique, l'étozoline, l'hydracarbazine, l'isosorbide, le mannitol, la métochalcone, la muzolimine, la perhexiline, le ticrynafène, le triamtérène, l'urée ou un de ses sels pharmaceutiquement acceptables.

17. Composition ou produit suivant la revendication 15, dans laquelle le diurétique est l'amiloride ou le bendrofluméthiazide.

18. Utilisation
(a) d'un premier composé, ledit premier composé étant l'atorvastatine ou un de ses sels pharmaceutiquement acceptables ; et
(b) d'un second composé, ledit second composé étant un diurétique ou un de ses sels pharmaceutiquement acceptables ;
dans la préparation d'un médicament destiné au traitement de l'angine de poitrine, de l'athérosclérose, de l'hypertension et de l'hyperlipidémie combinées et dans la prise en compte du risque cardiaque.

19. Utilisation suivant la revendication 18, dans laquelle lesdits premier et second composés sont en outre définis dans l'une quelconque des revendications 14 à 17.

20. Kit pour obtenir un effet thérapeutique chez un mammifère, comprenant :
a. une quantité d'atorvastatine ou d'un de ses sels pharmaceutiquement acceptables et un support ou diluant pharmaceutiquement acceptable dans une première forme posologique unitaire ;
b. une quantité d'un diurétique ou d'un de ses sels pharmaceutiquement acceptables, et un support ou diluant pharmaceutiquement acceptable dans une seconde forme posologique unitaire ; et
c. un moyen servant de récipient pour contenir lesdites première et seconde formes posologiques.

21. Kit suivant la revendication 20, dans lequel l'atorvastatine, l'effet thérapeutique et le diurétique sont en outre définis dans les revendications 14 à 17.

22. Utilisation d'un kit suivant les revendications 20 et 21, pour la préparation d'un médicament destiné au traitement de l'angine de poitrine, de l'athérosclérose, de l'hypertension et de l'hyperlipidémie combinées et dans la prise en compte du risque cardiaque.

23. Utilisation suivant la revendication 22, dans laquelle lesdits premier et second composés sont présents dans des formes posologiques distinctes et sont adaptés à l'administration simultanée ou séquentielle.
